# FASCICULE DE BREVET EUROPEEN

(11) **EP 2 398 404 B1**
(45) Date de publication et mention de la délivrance du brevet: **05.10.2016**
(21) Numéro de dépôt: 10704397.8
(22) Date de dépôt: 26.01.2010
(51) Int. Cl.: A61B 17/88, A61B 17/16, A61B 90/00, A61B 17/86

(54) **DISPOSITIF POUR FACILITER LA MISE EN PLACE DE VIS DANS LES TISSUS OSSEUX ET INSTRUMENTATION EN FAISANT APPLICATION, EN PARTICULIER POUR REALISER L'OSTEOSYNTHESE DE FRAGMENTS D'OS**
GERÄT ZUR ERLEICHTERUNG DER PLAZIERUNG VON SCHRAUBEN IM KNOCHENGEWEBE UND DAVON GEBRAUCH MACHENDE INSTRUMENTIERUNG, VOR ALLEM ZUR UMSETZUNG DER OSTHEOSYNTHESE VON KNOCHENFRAGMENTEN
APPARATUS FOR FACILITATING THE PLACEMENT OF SCREWS IN OSSEOUS TISSUES AND INSTRUMENTATION USING THIS APPARATUS, IN PARTICULAR FOR REALIZING OSTHEOSYNTHESIS OF FRAGMENTED BONES

(30) Priorité: 19.02.2009 FR 0900757
(43) Date de publication de la demande: 28.12.2011
(73) Titulaire: BIOTECH ORTHO, 13300 Salon-de-Provence (FR)
(72) Inventeur: IMPELLIZZERI, Frédéric, 13300 Salon de Provence (FR)
(74) Mandataire: Marek, Pierre
(86) Numéro de dépôt international: PCT/FR2010/000063
(87) Numéro de publication internationale: WO 2010/094846

(56) Documents cités:
- EP-A- 1 442 713
- WO-A-2008/085985
- WO-A-2008/140748

## Description

La présente invention concerne un dispositif facilitant la mise en place de vis dans les tissus osseux, en particulier en chirurgie orthopédique. Elle vise également l'instrumentation opératoire faisant application du dispositif selon l'invention.

Le protocole opératoire connu pour la pose de vis dans les tissus osseux, par exemple pour réduire une fracture par ostéosynthèse, et en particulier pour la pose de vis canulées, est le suivant :
- perforer les fragments d'os à l'aide d'une broche, généralement lisse et pointue, mue en rotation par un moteur équipé d'un embout passe-broches ;
- remplacer l'embout passe-broches par un embout à enclenchement rapide du type connu sous le nom d' « A.O. Synthes », ou par un embout universel type mandrin 3 mors,
- utiliser un foret canulé et gradué qui a pour fonction de préparer le logement de la vis et de mesurer la profondeur du trou, afin de déterminer la longueur de la vis à utiliser,
- utiliser une fraise canulée pour effectuer le chambrage de la tête de vis,
- retirer la fraise et placer la vis.

Une telle intervention, qui nécessite la manipulation successive de plusieurs instruments peut, selon les cas de réduction de fracture à traiter, s'avérer relativement longue et complexe.

Le protocole opératoire exposé ci-dessus peut être mis en oeuvre au moyen du système décrit dans le document EP 1 442 713, lequel divulgue un outil de perçage pour un os, en particulier pour le fémur proximal, comprenant une tige longitudinale comportant une partie antérieure de perçage munie d'un foret, une partie postérieure de serrage permettant son accouplement à un dispositif d'entraînement en rotation et une partie intermédiaire munie d'une ouverture longitudinale, ladite tige présentant un perçage axial traversant pour la réception d'une broche du type fil de Kirschner, cette ouverture communiquant avec le perçage axial et étant agencée pour constituer une fenêtre de contrôle permettant d'apercevoir l'extrémité postérieure de ladite broche, pendant le processus de perçage. Ce système présente les inconvénients soulignés précédemment.

On cite encore les documents WO-2008/140748 et WO-2008/085985, pour illustrer l'état de la technique.

On a proposé, dans le domaine de la chirurgie orthopédique (WO-2008/036.309), un instrument permettant de forer un passage de guidage dans un os, tout en mesurant la profondeur de ce passage au fur et à mesure de son exécution, afin de déterminer la longueur des vis à utiliser.

Cet instrument est constitué d'un foret non canulé, qui comprend une portion de coupe et une portion d'arbre muni d'une pluralité d'anneaux. La jauge comprend également un manche de perçage et une unité de lecture ayant un composant femelle et un composant male ayant une extrémité avant et une extrémité arrière et une pluralité d'anneaux sur l'extrémité arrière.

Un inconvénient majeur d'un tel instrument est qu'il ne peut servir à faciliter et choisir le bon positionnement des vis d'ostéosynthèse, telles que des vis de compression, puisqu'il n'y a pas de contrôle préalable possible de la direction de ladite vis, comme cela se fait dans le cas de l'utilisation de la broche guide, de sorte que leur insertion peut s'avérer défectueuse.

Un autre inconvénient important de l'instrument décrit dans le document WO-2008/036.309 est qu'il ne permet pas une réduction notable du temps de pose des vis orthopédique en raison du fait que les opérations de perçage et de chambrage doivent être exécutés séparément, par des instruments différents. Le temps pendant lequel les patients sont sous anesthésie est d'autant plus long, ce qui constitue une contrainte, aussi bien pour le chirurgien que pour les patients.

Dans le document US 2006/0.184.174, est décrit un foret réglable pour l'utilisation dans l'implantation de vis orthopédiques, telles que des vis de compression réglables ayant une tête réglable séparée. Le foret réglable comprend un foret principal et un foret de fraisage qui encercle le premier foret. Le foret de fraisage est configuré pour glisser de manière sélective le long du foret principal afin de l'utiliser en sélectionnant une profondeur de forage. Le foret de fraisage est associé à un membre d'arrêt qui est configuré pour verrouiller sélectivement le foret de fraisage à l'une des positions sélectionnée le long du foret principal afin d'établir ainsi une profondeur de forage. La profondeur du pré-perçage correspond à la taille de la vis sélectionnée par le chirurgien.

Cet instrument permet de forer des passages dans une matière osseuse en fonction de la longueur des vis que le chirurgien souhaite utiliser. Or, dans la majorité des cas de réduction de fracture, il n'est pas possible de déterminer la longueur des vis à utiliser avant de connaître la profondeur des pré-perçages exécutés dans les fragments d'os à réunir.

D'autre part, cet instrument présente également l'inconvénient précédemment souligné de ne pas prédire le positionnement des vis.

Dans certains cas, les praticiens préfèrent délaisser ce type de protocole opératoire au profit de l'utilisation de vis auto-perforantes, et ainsi s'affranchir du temps opératoire correspondant au perçage.

Un but de la présente invention est de remédier à ces inconvénients en apportant aux chirurgiens une solution fiable permettant une pose de vis d'ostéosynthèse plus précise et plus rapide.

Selon l'invention, ce but est atteint grâce à un dispositif polyvalent, permettant d'effectuer toutes les étapes de la mise en place de vis orthopédiques dans les tissus osseux, en particulier pour réaliser l'ostéosynthèse de fragments d'os, selon la revendication 1.

Avec l'utilisation du dispositif selon l'invention, le protocole de pose de vis orthopédiques est le suivant :
- introduire la broche à l'intérieur du corps creux de sorte que la partie distale de celle-ci dépasse de la partie distale dudit corps ;
- à l'aide du mandrin d'un appareil d'entraînement en rotation et préférentiellement sur un passe-broches, serrer simultanément le corps et la broche ;
- forer un passage dans les tissus osseux par l'intermédiaire de la broche, sous contrôle radiologique ;
- interrompre la rotation de l'ensemble lorsque le passage réalisé a atteint la profondeur souhaitable, c'est-à-dire lorsque l'extrémité distale de la broche a atteint la paroi corticale opposée, et desserrer le mandrin d'entraînement ;
- faire coulisser le corps creux le long de la broche jusqu'à ce que l'extrémité distale de celui-ci constituée par l'extrémité de la fraise vienne en appui contre la corticale supérieure de l'os;
- sur la fenêtre de lecture, lire la longueur de la vis à utiliser ;
- positionner et serrer le mandrin du dispositif d'entraînement de sorte à entraîner uniquement ledit corps creux en rotation ;
- fraiser la corticale supérieure de l'os afin de créer le chambrage de réception de la tête de vis ;
- relâcher le mandrin du dispositif d'entraînement et retirer le corps creux, en laissant la broche en position dans l'os;
- placer une vis auto-taraudeuse, comportant un alésage axial et présentant la longueur adéquate, autour de la broche et la faire coulisser sur celle-ci de sorte à l'amener au contact de la matière osseuse ;
- à l'aide d'un tournevis comportant également un alésage axial permettant son coulissement et sa rotation le long et autour de la broche, exécuter le vissage de la vis dans la matière osseuse.

Le dispositif selon l'invention procure plusieurs avantages intéressants. Il permet notamment :
- une estimation précise de la longueur des vis orthopédiques à utiliser,
- une mise en place précise grandement facilitée,
- l'utilisation d'un unique instrument, et donc une réduction des sources d'erreurs,
- une diminution du temps de pose des vis orthopédiques, et donc une réduction du temps d'anesthésie.

La portion extrême de la partie proximale du corps creux est munie de fentes longitudinales, par exemple de quatre fentes longitudinales également espacées. Cet agencement permet de serrer le corps sur la broche et d'assurer l'entraînement en rotation simultané dudit corps et de ladite broche.

Selon un mode d'exécution préféré, la portion extrême fendue de la partie proximale du corps présente un diamètre plus réduit que celui de la portion attenante non fendue de ladite partie proximale.

Selon un mode d'exécution avantageux, le corps creux, cylindrique ou approximativement cylindrique, comprend une partie centrale aplatie munie de la fenêtre de lecture graduée.

Selon un mode d'exécution préféré, la fenêtre de lecture est constituée d'une fente rectiligne longitudinale ménagée dans l'une des faces de la partie centrale aplatie du corps, et est munie, sur au moins l'un de ses bords, d'une graduation.

Cette graduation est, par exemple, constituée, sur l'un des bords de la fente, de graduations unitaires et sur l'autre bord de ladite fente, par des repères numériques multiples de 5.

Selon un autre mode d'exécution avantageux, les deux faces opposées de la portion centrale aplatie du corps sont munies d'une fente longitudinale graduée sur l'un des bords de ladite fente, afin que la lecture de la profondeur du trou soit possible quelle que soit la position de l'instrument.

Selon un mode de réalisation avantageux, la broche est munie, dans sa portion intermédiaire, à une distance prédéterminée de sa pointe active, d'un moyen de repérage pouvant être constitué par un anneau ou une zone colorée, ou préférentiellement, par un anneau ou une zone directement gravés dans le matériau constituant la broche.

Les buts, caractéristiques et avantages ci-dessus, et d'autres encore, ressortiront mieux de la description ci-dessous et des dessins annexés dans lesquels :

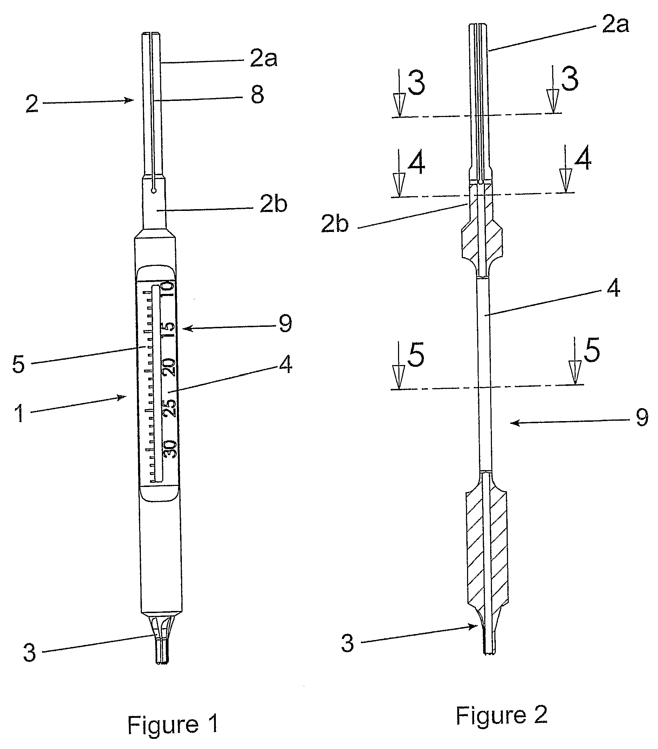

La figure 1 est une vue de face d'un exemple d'exécution du dispositif selon l'invention.
La figure 2 est une vue de coté et en coupe axiale du corps creux.

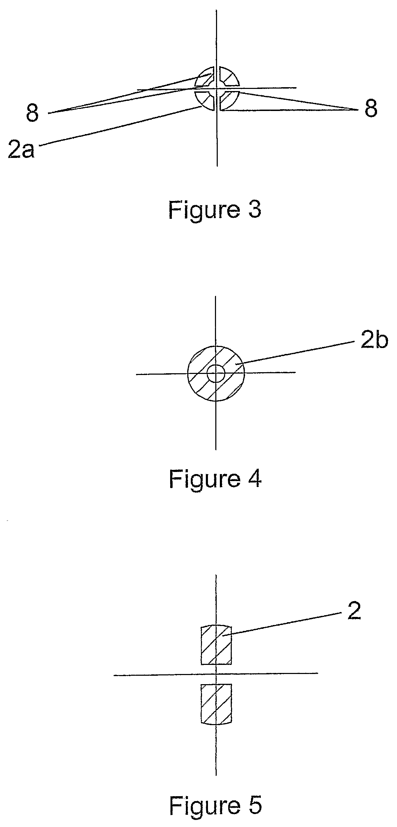

Les figures 3, 4 et 5 sont des vues en coupe radiale et à échelle agrandie selon les lignes 3-3, 4-4, 5-5, respectivement, de la figure 2.

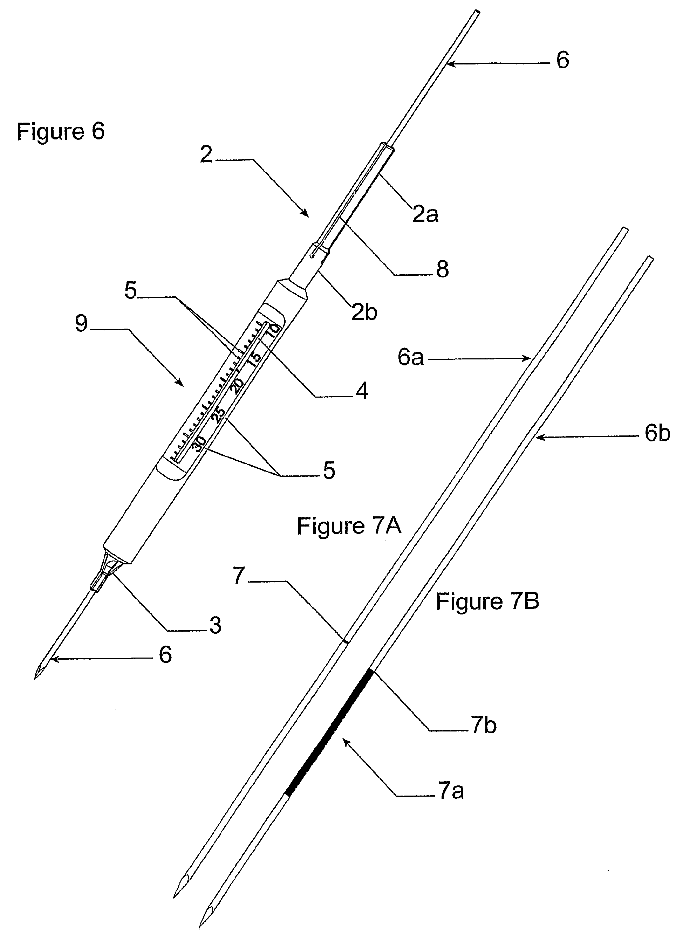

La figure 6 est une vue en perspective montrant le dispositif montré avec une aptitude de coulissement sur une broche perforante.
Les figures 7A et 7B illustrent deux modes d'exécution de la broche perforante.

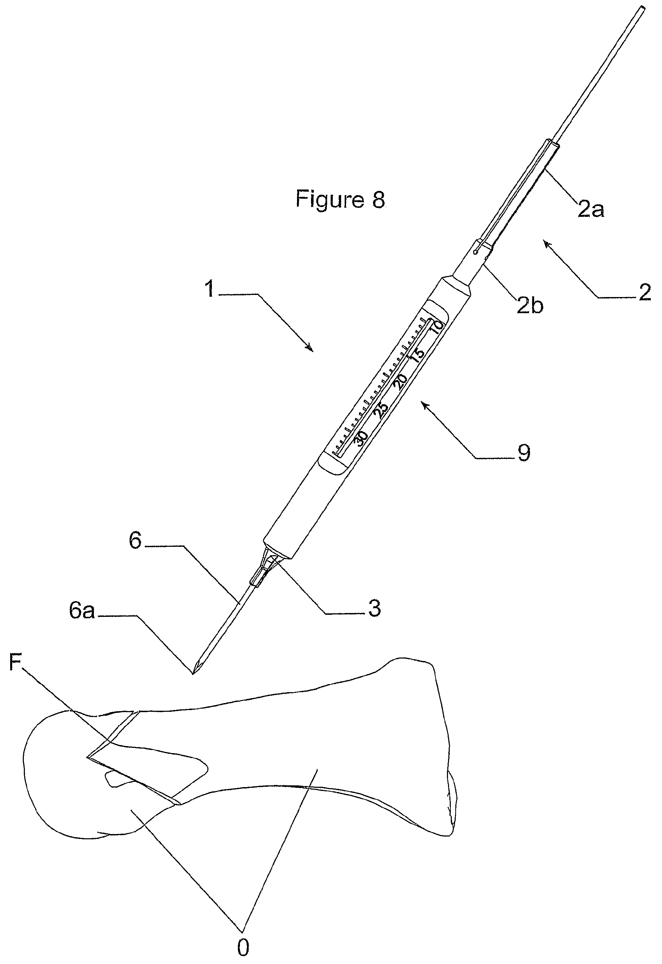

La figure 8 illustre la broche en place dans le corps creux lors de l'étape de perforation de l'os du protocole de vis osseuses.

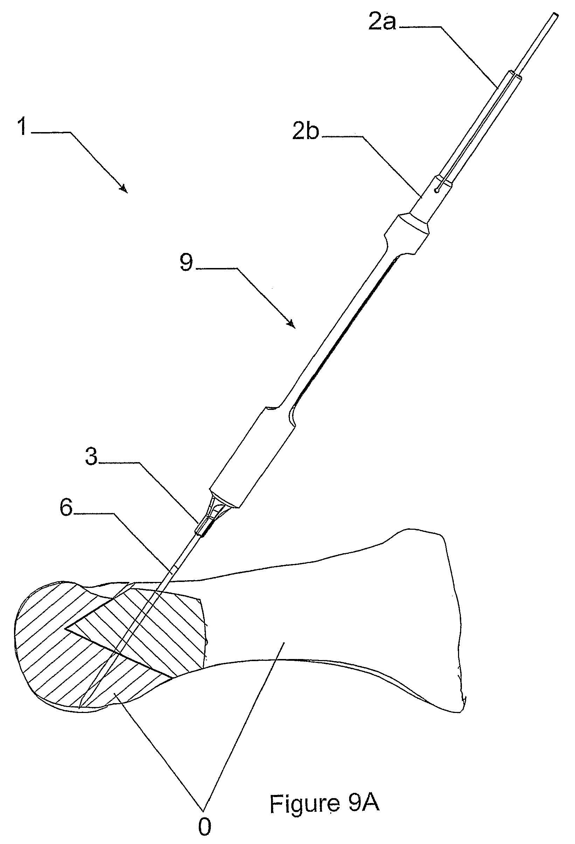

La figure 9A est une vue avec coupe partielle montrant la broche en place dans les différents fragments d'os.

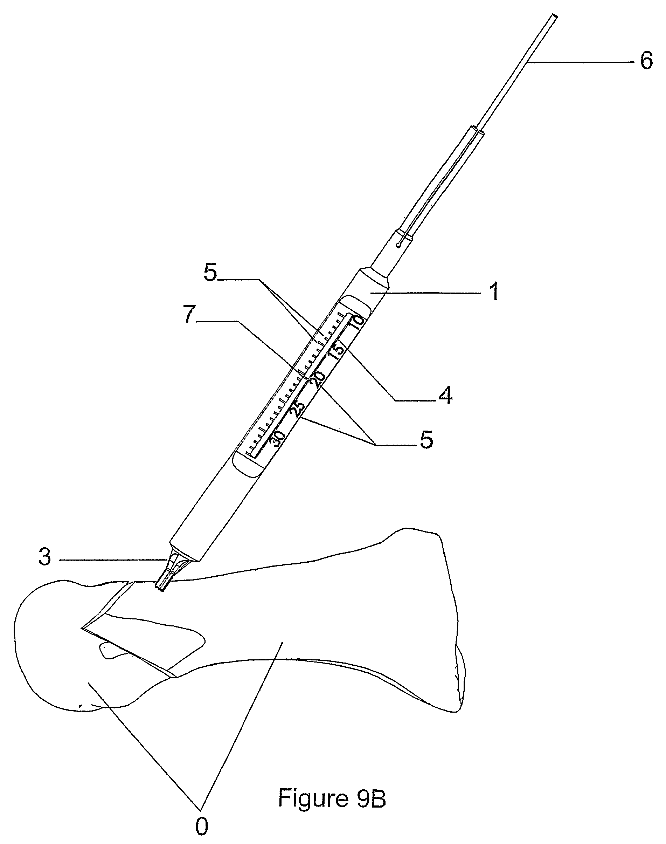

La figure 9B représente l'étape de lecture de la profondeur du trou du protocole de mise en place de vis osseuses.

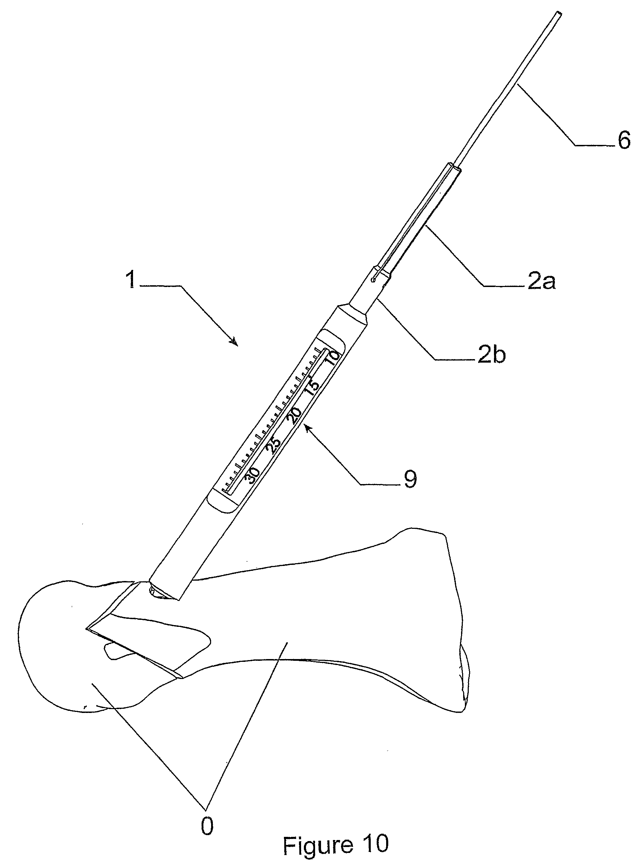

La figure 10 est une vue illustrant l'étape de fraisage du protocole de mise en place de vis osseuses.

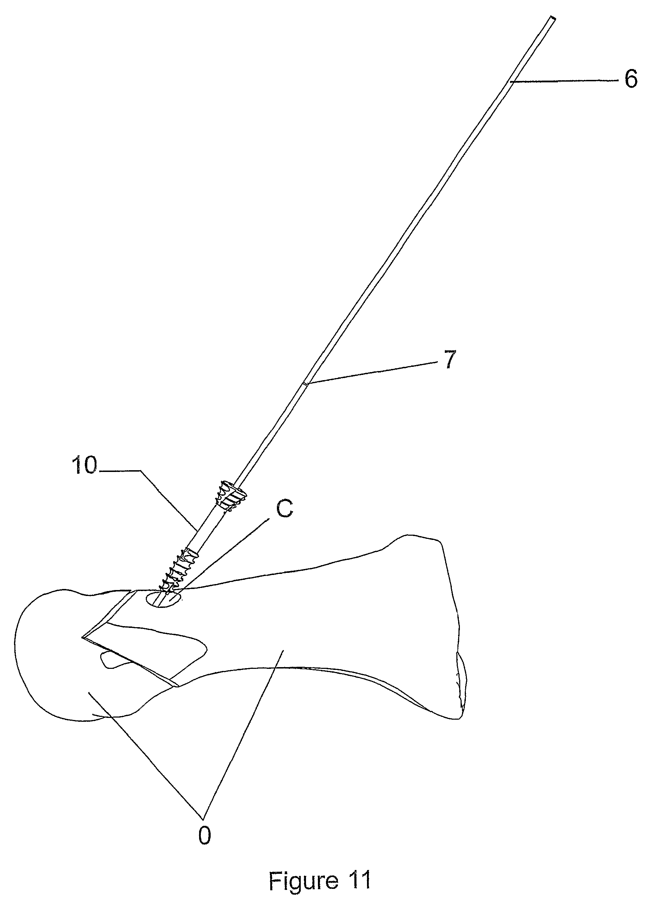

La figure 11 est une vue représentant une vis auto-perforante en place sur la broche.

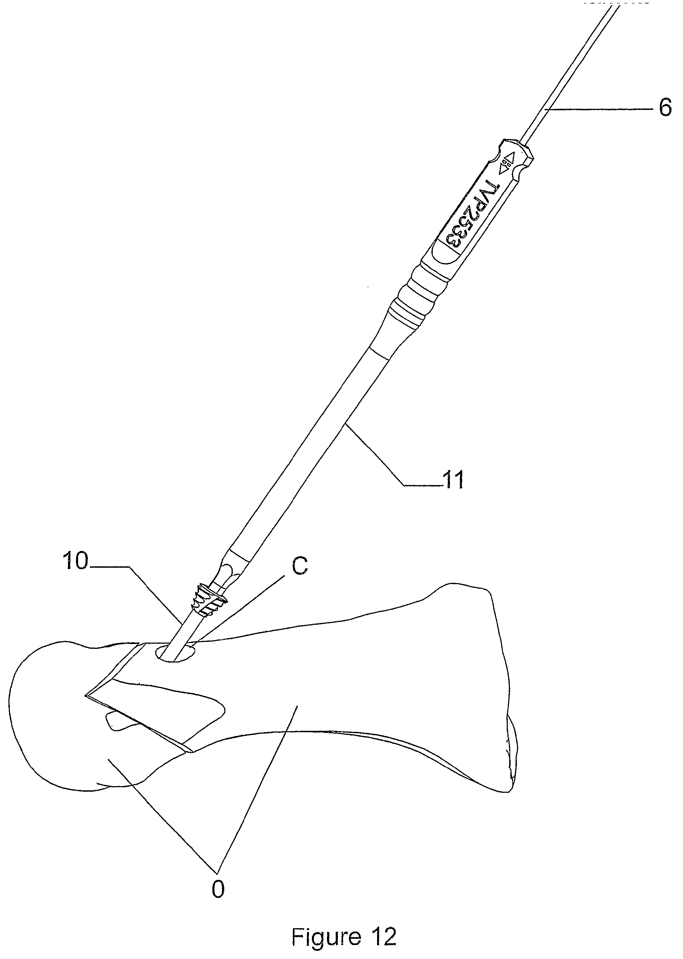

La figure 12 est une vue illustrant la pose d'une vis auto-perforante dans l'os à l'aide d'un tournevis.

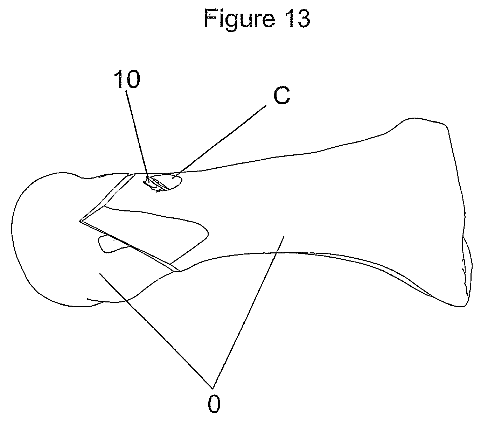

La figure 13 montre une vis orthopédique en place dans l'os.

On se réfère auxdits dessins pour décrire un exemple intéressant quoique nullement limitatif, de réalisation du dispositif de mise en place de vis dans les tissus osseux selon l'invention.

Le dispositif selon l'invention comprend principalement:
- d'une part, un corps creux allongé 1 comportant :
   - une partie proximale 2 permettant sa fixation à un mandrin passe-broches ou autre dispositif d'entraînement en rotation,
   - une partie distale munie d'une fraise de forme 3 permettant de réaliser le chambrage de réception de la tête des vis,
   - une partie centrale munie d'une fenêtre de lecture longitudinale 4 pourvue d'une graduation 5 et, d'autre part,
- une broche avec pointe trocart 6 présentant un diamètre permettant son coulissement axial dans l'alésage du corps creux 1 et comportant, dans sa partie intermédiaire, un moyen de repérage 7 susceptible d'être amené en regard de la fenêtre de lecture 4 dudit corps ;
la partie proximale 2 du corps 1 étant agencée pour permettre soit l'entraînement en rotation simultané du corps 1 et de la broche 6 logée dans celui-ci, soit l'entraînement dudit corps seulement.

Selon l'exemple illustré, la portion extrême 2a de la partie proximale 2 du corps creux 1 est munie de fentes longitudinales 8, par exemple de quatre fentes longitudinales également espacées. Cet agencement permet de serrer le corps 1 sur la broche 6 et d'assurer l'entraînement en rotation simultané dudit corps et de ladite broche.

Selon l'exemple représenté, la partie proximale 2 du corps creux 1 est encore munie d'une portion 2b de raccordement de ladite portion d'extrémité 2a à la partie restante du corps creux.

Avantageusement, la paroi latérale de la portion extrême fendue 2a de la partie proximale du corps présente un diamètre inférieur au diamètre de la portion attenante non fendue 2b de ladite partie proximale. Cet agencement permettant de ressentir un point dur lors du passage d'une configuration à l'autre.

De même, la paroi latérale de la portion non fendue 2b du corps 1 présente un diamètre inférieur au reste dudit corps.

Selon le procédé de l'invention, le corps creux 1, cylindrique ou approximativement cylindrique, comprend une partie centrale aplatie 9 munie de la fenêtre de lecture 4 graduée.

Selon le mode d'exécution représenté, la fenêtre de lecture 4 est constituée d'une fente rectiligne longitudinale ménagée dans l'une des faces de la partie centrale aplatie 9 du corps 1, et est munie, sur au moins l'un de ses bords, d'une graduation 5.

Cette graduation 5 est, par exemple, constituée, sur l'un des bords de la fente, d'une graduation millimétrique, et, sur l'autre bord de ladite fente, par des repères numériques multiples de 5.

De préférence et avantageusement, les deux faces opposées de la portion centrale aplatie 9 du corps creux 1 sont munies d'une fente longitudinale graduée 4 sur l'un des bords de ladite fente, afin que la lecture de la profondeur du trou soit possible quelle que soit la position de l'instrument.

Selon l'exemple illustré, la broche 6 est munie, dans sa portion intermédiaire, à une distance prédéterminée de sa pointe active 6a, d'un repère annulaire 7, pouvant être constitué par un anneau coloré, ou préférentiellement, par un anneau directement gravé dans le matériau constituant la broche afin d'être visible à travers la fenêtre de lecture 4. Le chirurgien peut alors lire la position de l'anneau sur les graduations correspondantes et déterminer la longueur de la vis orthopédique à utiliser.

De préférence et avantageusement, la broche 6 est munie, dans sa portion intermédiaire, à une distance prédéterminée de sa pointe active 6a, d'une zone annulaire 7a, pouvant être constitué par un revêtement coloré, ou préférentiellement, être directement gravé dans le matériau constituant la broche, afin d'être visible à travers la fenêtre de lecture 4. Le chirurgien peut alors lire la position de la transition 7b entre zone gravée et zone neutre sur les graduations correspondantes, et déterminer la longueur de la vis orthopédique à utiliser.

La broche 6 présente préférentiellement une surface lisse sur toute sa longueur et son extrémité distale foreuse 6a est pointue.

Le corps 1 peut être exécuté en tout matériau de grade médical présentant la dureté nécessaire, tel que par exemple acier inoxydable, titane, ...

D'autre part, la broche 6 peut être exécutée en tout matériau bio compatible présentant la dureté requise, tel que par exemple acier inoxydable, titane, ...

L'instrumentation selon l'invention inclut des vis orthopédiques canulées 10, préférentiellement des vis de compression, présentent un alésage axial de diamètre permettant le coulissement et la rotation aisés, mais sans jeu excessif, desdites vis le long et autour de la broche 6.

D'autre part, cette instrumentation comprend aussi un tournevis 11 utilisé pour placer les vis orthopédiques 10 dans les pré perçages réalisés à l'aide du dispositif selon l'invention, est également muni d'un alésage axial de diamètre permettant le coulissement et la rotation aisés, mais sans jeu excessif, dudit tournevis le long et autour de la broche 6.

Selon le protocole opératoire de la mise en place de vis osseuses à l'aide du dispositif selon l'invention, il faut introduire la broche 6 à l'intérieur du corps creux 1 de sorte que la partie distale de celle-ci dépasse largement de la partie distale dudit corps, et on serre simultanément ledit corps et ladite broche à l'aide du mandrin d'un appareil d'entraînement en rotation.

Cette mise en place est surveillée sur un écran, par exemple un écran radiologique, sur lequel le chirurgien peut visualiser la progression de la broche 6 dans les tissus osseux, jusqu'à ce que la pointe active 6a ait traversé les fragments d'os O, la fracture F, et qu'elle arrive au contact de la surface intérieure de la corticale inférieure de l'os.

Lorsque la broche 6 atteint la position désirée, on arrête la rotation de l'ensemble corps 1-broche 6 et on desserre le mandrin de l'appareil d'entraînement en rotation.

Le corps 1 est alors libre de coulisser le long de la broche 6 jusqu'à ce que l'extrémité de celui-ci, constituée de la fraise 3, vienne en appui contre la corticale supérieure de l'os O.

Le chirurgien peut alors lire sur la fenêtre de lecture 4, la profondeur du pré perçage ainsi réalisé et donc déterminer, rigoureusement, la longueur de la vis à utiliser.

Le praticien va ensuite venir serrer uniquement le corps 1 en positionnant le mandrin de l'appareil d'entraînement en rotation au niveau de la portion 2b de la partie proximale 2 du corps 1. Le moteur entraîne en rotation uniquement ledit corps dont la fraise 3 va effectuer le chambrage C de réception de la tête de la vis 10.

Lorsque le fraisage est terminé, le corps 1 est retiré pour ne laisser en position dans l'os que la broche 6.

Le chirurgien place ensuite une vis auto-taraudeuse 10, comportant un alésage axial 10a et présentant la longueur adéquate, autour de la broche 6 et la fait coulisser sur celle-ci de sorte à l'amener au contact de la matière osseuse.

A l'aide d'un tournevis 11 comportant également un alésage axial 11 a permettant son coulissement et sa rotation, le long et autour de la broche 6, le praticien exécute le vissage de la vis orthopédique 10 dans la matière osseuse.

La vis est alors en place dans la matière osseuse, on peut donc retirer le tournevis 11 et la broche 6.

## Revendications

1. Dispositif pour faciliter la mise en place de vis dans les tissus osseux, en particulier pour réaliser l'ostéosynthèse de fragments d'os, comprenant:
- d'une part, un corps creux allongé (1) comportant :
- une partie proximale (2) permettant sa fixation à un mandrin ou autre dispositif d'entraînement en rotation,
- une partie distale munie d'une fraise (3) permettant de réaliser le chambrage de réception de la tête des vis,
- une partie centrale munie d'une fenêtre de lecture longitudinale (4) et,
- d'autre part,
- une broche perforeuse (6) présentant un diamètre permettant son coulissement axial dans l'alésage du corps creux (1),
**caractérisée en ce que** la fenêtre est pourvue d'une graduation (5) et **en ce que** ladite broche perforeuse (6) comporte, dans sa partie intermédiaire, un moyen de repérage (7) susceptible d'être amené en regard de la fenêtre de lecture (4) dudit corps ;
la partie proximale (2) du corps (1) comprenant une première portion de fixation formée par une portion d'extrémité (2a) munie de fentes longitudinales (8) permettant de serrer le corps creux de façon concentrique sur la broche et permettant l'entraînement en rotation simultané dudit corps creux (1) et de la broche (6) logée dans celui-ci, et comprenant une deuxième portion de fixation formée par une portion (2b) de raccordement de ladite portion d'extrémité (2a) à la partie restante du corps creux, permettant l'entraînement en rotation de ce dernier seulement.

2. Dispositif suivant la revendication 1, **caractérisé en ce que** le diamètre de la paroi latérale de la portion d'extrémité fendue (2a) est inférieur au diamètre de la paroi latérale de la portion attenante non fendue (2b) de la partie proximale (2) dudit corps creux (1).

3. Dispositif selon l'une quelconque des revendications 1 ou 2, dont le corps creux (1) présente une forme générale cylindrique ou approximativement cylindrique, **caractérisé en ce que** ledit corps présente une partie centrale aplatie (9) dont une face au moins est munie d'une fenêtre de lecture graduée (4).

4. Dispositif suivant la revendication 3, **caractérisé en ce que** les deux faces opposées de la partie centrale aplatie (9) du corps creux (1) sont munies d'une fenêtre de lecture graduée (4).

5. Dispositif selon l'une quelconque des revendications 1 à 4, **caractérisé en ce que** la fenêtre de lecture (4) ou chaque fenêtre de lecture, est constituée par une fente longitudinale ménagée dans la partie centrale (9) du corps creux (1) et munie d'une graduation (5) sur au moins l'un de ses bords.

6. Dispositif suivant la revendication 5, **caractérisé en ce que** ladite graduation comprend, d'une part, une graduation millimétrique répartie le long de l'un des bords de la fenêtre de lecture (4), et, d'autre part, des repères numériques multiples de 5, répartis le long du bord opposé de ladite fenêtre de lecture.

7. Dispositif selon l'une quelconque des revendications 1 à 6, **caractérisé en ce que** la broche (6) est munie, dans sa portion intermédiaire, à une distance prédéterminée de sa pointe active (6a), d'un repère annulaire (7), pouvant être constitué par un anneau coloré, ou préférentiellement, par un anneau directement gravé dans le matériau constituant la broche, ou par une zone annulaire 7a, pouvant être constituée par un revêtement coloré, ou préférentiellement, être directement gravé dans le matériau constituant la broche.

8. Instrumentation pour faciliter la mise en place de vis dans les tissus osseux, en particulier pour réaliser l'ostéosynthèse de fragments d'os, incluant un dispositif selon l'une quelconque des revendications 1 à 7, **caractérisée en ce qu'**elle comprend également des vis de compression ou autres vis orthopédiques (10) munies d'un alésage axial dont le diamètre permet leur coulissement et leur rotation aisés, mais sans jeu excessif, le long et autour de la broche (6).

9. Instrumentation selon la revendication 8, **caractérisée en ce qu'**elle comprend encore un tournevis (11) pourvu d'un alésage axial dont le diamètre permet son coulissement et sa rotation aisés, mais sans jeu excessif, le long et autour de la broche (6).

## Patentansprüche

1. Gerät zur Erleichterung der Platzierung von Schrauben in das Knochengewebe, insbesondere zur Umsetzung der Osteosynthese von Knochenfragmenten umfassend:
- erstens einen länglichen Hohlkörper (1), umfassend:
- einen proximalen Abschnitt (2) für seine Befestigung an einem Dom oder einer weiteren drehenden Antriebsvorrichtung,
- einen distalen Abschnitt mit einer Schneidevorrichtung (3) zur Realisierung einer Aufnahme zum Versenken des Schraubenkopfes,
- einen zentralen Abschnitt mit einem länglichen Sichtfenster (4) und,
- zweitens,
- einen Stanzstift (6) mit einem Durchmesser der es ermöglicht, axial in der Bohrung des Hohlkörpers (1) zu gleiten, **dadurch gekennzeichnet, dass** das Fenster mit einer Skala (5) versehen ist und wobei der besagte Stanzstift (6), in seinem Zwischenabschnitt, ein Lokalisierungsmittel (7) aufweist, das geeignet ist, gegenüber dem Sichtfenster (4) des Körpers geführt zu werden,
der proximale Abschnitt (2) des Körpers (1) ist mit einem ersten Befestigungsabschnitt durch einen Endabschnitt (2a) ausgebildet (2a), der längliche Schlitze (8) ausbildet, die eine konzentrische Klemmung des Hohlkörpers am Stanzstift und gleichzeitig den Drehantrieb des besagten Hohlkörpers (1) und des Stanzstiftes (6), der darin untergebracht ist sowie umfassend einen zweiten Befestigungsabschnitt ausgebildet durch einen Verbindungsabschnitt (2b) des genannten Befestigungsabschnittes(2a)ausgebildet an dem verbleibenden Teil des Hohlkörpers, der einen Drehantrieb des letzteren alleine ermöglicht.

2. Vorrichtung nach Anspruch 1, **dadurch gekennzeichnet, dass** der Durchmesser der Seitenwand des geschlitzten Endabschnitts (2a) geringer ist als der Durchmesser der Seitenwand des angrenzenden nicht geschlitzten Abschnitt (2b) des proximale Abschnitt (2) des Hohlkörpers (1).

3. Vorrichtung nach einem der Ansprüche 1 oder 2, bei dem der Hohlkörper (1) eine im Wesentlichen zylindrische oder annähernd zylindrische Form aufweist, **dadurch gekennzeichnet, dass** der besagte Hohlkörper einen zentralen abgeflachten Abschnitt (9) aufweist, dessen zumindest eine Seite mit einem Sichtfenster mit Skala (4) versehen ist.

4. Vorrichtung nach Anspruch 3. **dadurch gekennzeichnet, dass** die beidseitig gegenüberliegenden Seiten des abgeflachten zentralen Abschnitts (9) des Hohlkörpers (1) mit einem Sichtfenster (4) mit Skala versehen sind.

5. Vorrichtung nach einem der Ansprüche 1 bis 4, **dadurch gekennzeichnet, dass** das Sichtfenster (4) oder jedes der Sichtfenster durch einen Längsschlitz in dem zentralen Mittelabschnitt (9) des Hohlkörpers (1) gebildet wird und mit einer Skala (5) an mindestens einem seiner Seiten versehen ist.

6. Vorrichtung nach Anspruch 5. **dadurch gekennzeichnet, dass** die genannte Skala zum einen eine Millimeterskala umfasst, die entlang einer der Kanten des Sichtfensters (4) angebracht ist und zum anderen Leitzahlen als Vielfache von 5, entlang der gegenüberliegenden Kante des Sichtfensters aufgeteilt sind.

7. Vorrichtung nach einem der Ansprüche 1 bis 6, **dadurch gekennzeichnet, dass** der Dom (6) in seinem mittleren Abschnitt an einem vorbestimmten Abstand zu seiner Arbeitsspitze (6a), mit einer ringförmigen Markierung (7) ausgebildet ist, der durch einen farbigen Ring gebildet wird oder bevorzugt durch ein Ring direkt im Material des Doms eingraviert ist oder durch eine ringförmige Zone 7a, die durch eine gefärbte Beschichtung gebildet werden kann oder bevorzugt direkt in das Material des Doms eingraviert sein kann.

8. Instrumentierung zur Erleichterung der Platzierung von Schrauben in das Knochengewebe, insbesondere zur Umsetzung der Osteosynthese von Knochenfragmenten mit einer Vorrichtung nach einem der Ansprüche 1 bis 7. **dadurch gekennzeichnet, dass** sie auch Kompressionsschrauben oder andere orthopädische Schrauben (10), die mit einer axialen Bohrung versehen sind, umfasst, deren Durchmesser das Gleiten und ihre leichte Drehung ermöglicht, jedoch ohne übermäßiges Spiel entlang und um den Dom (6) herum.

9. Instrumentierung gemäß Anspruch 8, **dadurch gekennzeichnet, dass** sie auch einen Schraubendreher (11) umfasst, der eine axiale Bohrung aufweist, deren Durchmesser das Gleiten und eine leichte Drehung ermöglicht, jedoch ohne übermäßiges Spiel entlang und um den Dom (6) herum.

## Claims

1. Device for facilitating the placement of screws in osseous tissues, particularly for carrying out osteosynthesis of fragmented bones, comprising:
- on the one hand, an elongated hollow body (1) comprising:
- a proximal part (2) allowing it to be fixed to a mandrel or another rotary drive device;
- a distal part provided with a cutter (3) that allows the countersink to be produced for receiving the screw head;
- a central part provided with a longitudinal reading window (4); and
- on the other hand,
- a perforating spindle (6), the diameter of which allows it to axially slide inside the bore of the hollow body (1),
**characterised in that** the window is provided with a graduated scale (5) and **in that** said perforating spindle (6) comprises, in its intermediate part, a marking means (7) capable of being brought into the view of the reading window (4) of said body;
the proximal part (2) of the body (1) comprising a first fixing portion formed by an end portion (2a) provided with longitudinale slots (8) allowing the hollow body to be concentrically clamped on the spindle and allowing the simultaneous rotary drive of said hollow body (1) and of the spindle (6) housed therein, and comprising a second fixing portion formed by a portion (2b) for connecting said end portion (2a) to the remaining part of the hollow body, allowing the rotary drive of said hollow body only.

2. Device according to claim 1, **characterised in that** the diameter of the side wall of the slotted and portion (2a) is less than the diameter of the side wall of the adjoining non-slotted portion (2b) of the proximal part (2) of said hollow body (1).

3. Device according to any one of claims 1 to 2, whereon the hollow body (1) has a general cylindrical or relatively cylindrical shape, **characterised in that** said body has a flattened central part (9), at least one face of which is provided with a graduated reading window (4).

4. Device acoording to claim 3, **characterised in that** the two opposite feces of the flattened central part (9) of the hollow body (1) are provided with a graduated reading window (4).

5. Device according to any one of claims 1 to 4, **characterised in that** the reading window (4), or each reading window, is formed by a longitudinal slot arranged in the central part (9) of the hollow body (1) and provided with a graduated scale (5) on at least one of its edges.

6. Device acoording to claim 5, **characterised in that** said graduated scale comprises, on the one hand, a millimetric graduated scale distributed along one of the edges of the reading window (4) and, on the other hand, digital references in multiples of five distributed along the opposite edge of said reading window.

7. Device according to any one of claims 1 to 6, **characterised in that** the spindle (6) is provided, in its intermediate portion at a predetermined distance from its active tip (6a), with an annular reference marker (7) that can be formed by a coloured ring or, preferably, by a ring directly etched into the material forming the spindle, or by an annular zone 7a that can be formed by a coloured coating or, preferably, can be directly etched into the material forming the spindle.

8. Instrumentation for facilitating the placement of screws in osseous tissues, particularly for carrying out osteosynthesis of fragmented bones, comprising a device according to any one of claims 1 to 7, **characterised in that** it further comprises compression screws or other orthopaedic screws (10) provided with an axial bore, the diameter of which allows them to easily slide and rotate, without excessive play, along and about the spindle (6).

9. Instrumentation according to claim 8, **characterised in that** it further comprises a screwdriver (11) provided with an axial bore, the diameter of which allows it to easily slide and rotate, without excessive play, along and about the spindle (6).
